(19) Europäisches Patentamt
European Patent Office
Office européen des brevets

(11) Veröffentlichungsnummer: **0 309 408**
**A1**

(12) **EUROPÄISCHE PATENTANMELDUNG**

(21) Anmeldenummer: **88810636.6**

(22) Anmeldetag: **16.09.88**

(51) Int. Cl.[4]: **C 07 C 127/22**
C 07 C 87/60, C 07 C 79/12,
A 01 N 47/34

(30) Priorität: **25.09.87 CH 3715/87**
**21.07.88 CH 2793/88**

(43) Veröffentlichungstag der Anmeldung:
**29.03.89 Patentblatt 89/13**

(84) Benannte Vertragsstaaten:
**BE CH DE ES FR GB IT LI NL**

(71) Anmelder: **CIBA-GEIGY AG**
**Klybeckstrasse 141**
**CH-4002 Basel (CH)**

(72) Erfinder: **Drabek, Jozef, Dr.**
**Benkenstrasse 12**
**CH-4104 Oberwil (CH)**

(54) **N-Benzoyl-N'-2,4-difluor-3,5-dihalogenphenylharnstoffe, ihre Herstellung und Verwendung bei der Kontrolle von Schädlingen.**

(57) Neue substituierte N-Benzoyl-N'-2,4-difluor-3,5-dihalogenphenylharnstoffe der Formel I

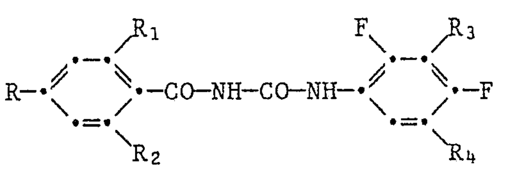

(I),

worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom
stehen, Verfahren und Zwischenprodukte zu ihrer Herstellung, ihre Verwendung in der Schädlingsbekämpfung und Schädlingsbekämpfungsmittel, welche eine Verbindung der Formel I als Wirkstoff enthalten, werden offenbart. Bevorzugtes Anwendungsgebiet ist die Kontrolle von Schädlingen an Tieren und Pflanzen.

EP 0 309 408 A1

**Beschreibung**

**N-Benzoyl-N′-2,4-difluor-3,5-dihalogenphenylharnstoffe, ihre Herstellung und Verwendung bei der Kontrolle von Schädlingen**

Die vorliegende Erfindung betrifft neue substituierte N-Benzoyl-N′-2,4-difluor-3,5-dihalogen-phenylharnstoffe, Verfahren und Zwischenprodukte zu ihrer Herstellung, Schädlingsbekämpfungsmittel, die diese Verbindungen enthalten, sowie ihre Verwendung bei der Kontrolle von Schädlingen.

Die erfindungsgemässen Verbindungen entsprechen der Formel I

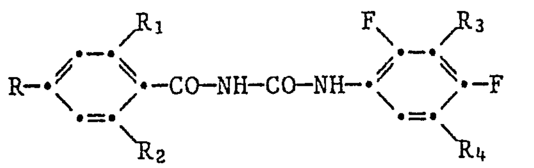

(I),

worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom
stehen.

Unter den Verbindungen der Formel I stehen diejenigen im Vordergrund, in denen R für Wasserstoff; $R_1$ für Wasserstoff, Fluor oder Chlor; $R_2$ für Fluor oder Chlor; $R_3$ für Brom und $R_4$ für Chlor oder Brom; oder $R_3$ für Chlor und $R_4$ für Brom stehen.

Dabei sind diejenigen Verbindungen der Formel I bevorzugt, in denen R für Wasserstoff; $R_1$ für Wasserstoff oder Fluor; $R_2$ für Fluor oder Chlor; $R_3$ für Brom und $R_4$ für Chlor; oder $R_3$ für Chlor und $R_4$ für Brom stehen.

Die erfindungsgemässen Verbindungen können nach an sich bekannten Verfahren hergestellt werden. Solche Verfahren sind u.a. in den DE-OS 21 23 236, 26 01 780 und 32 40 975 beschrieben. So können die Verbindungen der Formel I z.B. erhalten werden, indem man

a) ein Anilin der Formel II

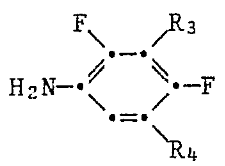

(II)

mit einem Benzoylisocyanat der Formel III

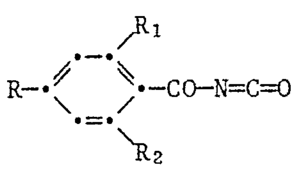

(III),

oder

b) ein Isocyanat der Formel IV

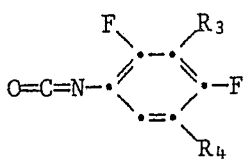

(IV)

mit einem Benzamid der Formel V

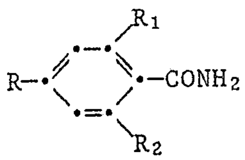

(V),

oder

c) ein Anilin der Formel II mit einem Urethan der Formel VI

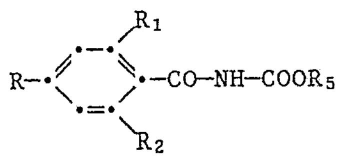

(VI)

umsetzt. In den Formeln II, III, IV, V und VI haben R, $R_1$, $R_2$, $R_3$ und $R_4$ die für Formel I angegebene Bedeutung und $R_5$ steht für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylole, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis +200°C, vorzugsweise zwischen 0 und +100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis +150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit einem Anilin der Formel II, werden Temperaturen zwischen etwa +60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III bis VI sind bekannt oder können analog bekannten Verfahren hergestellt werden.

Bei den Ausgangsstoffen der Formel II handelt es sich um neue Verbindungen, die ebenfalls einen Gegenstand der vorliegenden Erfindung bilden. Die Verbindungen der Formel II können in an sich bekannter Weise z.B. dadurch hergestellt werden, dass man das Nitrobenzol der Formel VII

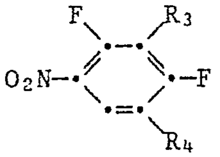

(VII)

katalytisch hydriert. Dabei haben $R_3$ und $R_4$ die für Formel I angegebene Bedeutung. Als Katalysatoren kommen die üblichen Hydrierungskatalysatoren in Betracht wie z.B. Platin, Palladium, Rhenium, Rheniumoxide oder Raney-Nickel (vgl. J. org. Chem. 29, 1964). Die Ueberführung der $NO_2$- in die $NH_2$-Gruppe kann auch durch übliche chemische Reduktion erfolgen z.B. mit Eisen oder Zinnchlorid in salzsaurem Medium.

Die Nitrobenzole der Formel VII sind neu, bilden ebenfalls einen Gegenstand der vorliegenden Erfindung und können nach einem im Prinzip bekannten Verfahren hergestellt werden, indem z.B. ein Nitrobenzol der Formel VIIIa-VIIIc

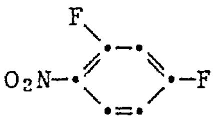

(VIIIa)

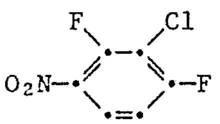

(VIIIb) oder

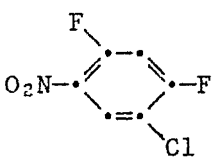

(VIIIc)

in üblicher Weise mit elementarem Brom gegebenenfalls in Gegenwart eines Katalysators, wie z.B. $FeCl_3$, bei +70 bis 200°C, vorzugsweise +100 bis 170°C und gegebenenfalls in einem Lösungsmittel wie z.B. einem perhalogenierten Kohlenwasserstoff bromiert.

Die Nitrobenzole der Formeln VIIIa bis VIIIc sind bekannt und können nach in Prinzip bekannten Verfahren hergestellt werden.

In der veröffentlichten europäischen Patentanmeldung 0 052 833 werden u.a. N-Halogenbenzoyl-N'-(2,4-difluor-3,5-dichlorphenyl)-harnstoffe als Insektizide mit larvizider Wirkung beschrieben.

Ueberraschenderweise wurde gefunden, dass die erfindungsgemässen Verbindungen der Formel I bei günstiger Warmblüter- und Pflanzenverträglichkeit stärkere Wirkstoffe bei der Kontrolle von Schädlingen sind. So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Kontrolle von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Insbesondere zeichnen sich die erfindungsgemässen Verbindungen durch eine ausgezeichnete larvizide Wirkung bei Spodoptera littoralis und vor allem bei Heliothis virescens aus.

Die gute pestizide Wirkung der erfindungsgemässen Verbindungen der Formel I entspricht einer Abtötungsrate (Mortalität) von mindestens 50-60 % der erwähnten Schädlinge.

Die gute pestizide Wirkung der erfindungsgemässen Verbindungen der Formel I entspricht einer

Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen, inerten, pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die einen Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. Zwischenprodukte

1.1.1. Nitrobenzole

1.1.1.1. 2,4-Difluor-3-chlor-5-brom-nitrobenzol

19,2 g 2,4-Difluor-3-chlor-nitrobenzol und 3 g wasserfreies $FeCl_3$ werden auf + 140° C erhitzt, unter Rühren tropfenweise mit 5,1 ml Brom versetzt und 48 Stunden lang bei dieser Temperatur nachgerührt. Das abgekühlte Reaktionsgemisch wird in Dichlormethan aufgenommen und mit Wasser gewaschen. Die organische Phase wird abgetrennt, über Natriumsulfat getrocknet und eingeengt. Der Rückstand wird an einer Kieselgelsäule mit Hexan/Dichlormethan (10:1) als Laufmittel gereinigt. Die Titelverbindung der Formel

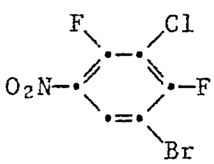

(Verb. Nr. 1.1.1.1.)

liegt als braunes Oel vor; Brechungsindex $n_D^{25}$: 1,5720.

Auf analoge Weise werden die folgenden Verbindungen hergestellt:

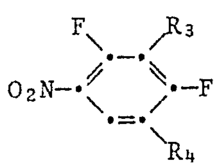

| Verb. Nr. | $R_3$ | $R_4$ | phys. Daten |
|---|---|---|---|
| 1.1.1.2. | Br | Cl | |
| | Br | Br | |

1.1.2. Aniline

1.1.2.1. 2,4-Difluor-3-chlor-5-bromanilin

7,6 g 2,4-Difluor-3-chlor-5-brom-nitrobenzol werden in 80 ml Tetrahydrofuran gelöst und bei +20 bis 25°C während 4 Stunden in Gegenwart von 1,7 g 5%iger Rhenium-Kohle hydriert (Wasserstoffaufnahme: 1,88 l). Das Reaktionsgemisch wird filtriert, das Lösungsmittel abdestilliert und der Rückstand in Hexan suspendiert und abgenutscht. Die Titelverbindung der Formel

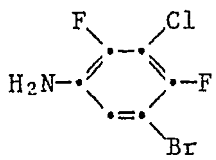

(Verb. Nr. 1.1.2.1.)

liegt in Form farbloser Kristalle vor; Smp. 86-88°C.

Auf analoge Weise werden die folgenden Verbindungen hergestellt:

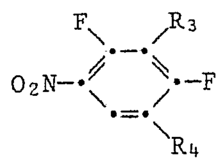

| Verb. Nr. | $R_3$ | $R_4$ | phys. Daten |
|---|---|---|---|
| 1.1.2.2. | Br | Cl | |
| | Br | Br | |

1.2. Endprodukte

1.2.1. N-(2,6-Difluorbenzoyl)-N'-(2,4-difluor-3-chlor-5-bromphenyl)-harnstoff

5 g 2,4-Difluor-3-chlor-5-bromanilin, gelöst in 50 ml trockenem Toluol, werden bei Raumtemperatur unter Rühren tropfenweise mit 3,75 g 2,6-Difluorbenzoylisocyanat versetzt und 5 Stunden lang nachgerührt. Anschliessend werden etwa 75 % des Lösungsmittels am Rotationsverdampfer entfernt. Der Rückstand wird mit Hexan verdünnt und der entstandene Niederschlag abgenutscht, mit Hexan gewaschen und im Vakuum getrocknet. Die Titelverbindung der Formel

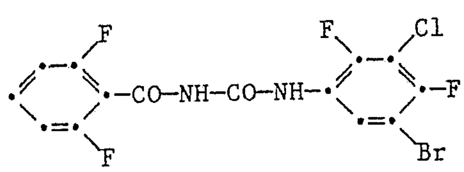

(Verb. Nr. 1.2.1.)

liegt in Form farbloser Kristalle vor; Smp. 225-227°C.

Auf analoge Weise werden die folgenden Verbindungen hergestellt:

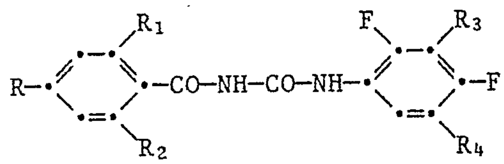

| Verb. Nr. | R | $R_1$ | $R_2$ | $R_3$ | $R_4$ | phys. Daten |
|---|---|---|---|---|---|---|
| 1.2.2. | H | H | Cl | Cl | Br | Smp. 230-232°C |
| 1.2.3. | F | F | F | Cl | Br | Smp. 220-221°C |
| | H | F | F | Br | Cl | |
| | H | H | F | Cl | Br | |
| | H | F | Cl | Cl | Br | |
| | H | Cl | Cl | Cl | Br | |
| | H | F | $OCH_3$ | Cl | Br | |
| | H | $OCH_3$ | $OCH_3$ | Cl | Br | |
| | H | H | $OCH_3$ | Cl | Br | |
| | H | H | Cl | Br | Cl | |
| | H | H | F | Br | Cl | |
| | H | F | Cl | Br | Cl | |
| | H | Cl | Cl | Br | Cl | |
| | H | F | $OCH_3$ | Br | Cl | |
| | H | $OCH_3$ | $OCH_3$ | Br | Cl | |
| | H | H | $OCH_3$ | Br | Cl | |
| | F | F | F | Br | Cl | |
| | H | F | F | Br | Br | |
| | H | H | Cl | Br | Br | |

Beispiel 2: Formulierungen von Wirkstoffen der Formel I gemäss Herstellungsbeispiel 1.2.

(% = Gewichtsprozent)

2.1. Emulsions-Konzentrate

| | a) | b) |
|---|---|---|
| Wirkstoff gemäss dem Herstellungsbeispiel 1.2. | 10 % | 25 % |
| Ca-Dodecyl-benzolsulfonat | - | 5 % |
| Ricinusöl-poly-äthylenglyko-läther (36 Mol AeO) | 25 % | 5 % |
| Butanol | 15 % | - |
| Xylolgemisch | - | 65 % |
| Essigester | 50 % | - |

Aus solchen Konzentraten können durch Verdünnen mit Wasser Emulsionen jeder gewünschten Konzentration hergestellt werden.

2.2. Lösungen

| | a) | b) |
|---|---|---|
| Wirkstoff gemäss dem Herstellungsbeispiel 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190° C) | - | 74 % |

Die Lösungen sind zur Anwendung in Form kleinster Tropfen geeignet.

2.3. Granulate

| | a) | b) |
|---|---|---|
| Wirkstoff gemäss dem Herstellungsbeispiel 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4. Extruder-Granulat

Wirkstoff gemäss dem Herstellungsbeispiel 1.2. 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

Wirkstoff gemäss dem Herstellungsbeispiel 1.2. 3 %
Polyäthylenglykol (MG 200) 3 %
Kaolin 94 %

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.6. Stäubemittel

| | a) | b) | c) | d) |
|---|---|---|---|---|
| Wirkstoff gemäss dem Herstellungsbeispiel 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

2.7. Spritzpulver

| | a) | b) | c) |
|---|---|---|---|
| Wirkstoff gemäss dem Herstellungsbeispiel 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8. Suspensions-Konzentrat

Wirkstoff gemäss dem Herstellungsbeispiel 1.2. 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %

Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss Beispiel 1.2. zeigen gute Wirkung im obigen Test.

3.2. Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 1.2. zeigen in diesem Test gute Wirkung gegen Lucilia sericata.

3.3. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1.2. zeigen gute Wirkung in diesem Test.

3.4. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss Beispiel 1.2. zeigen 100%ige Wirkung.

3.5. Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 0,75 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1.2. zeigen bei Spodoptera und bei Heliothis nach 2 und 5 Tagen 80-100%ige Wirkung (Mortalität).

3.6. Frassgift-Wirkung auf Plutella xylostella-Larven ($L_2$)

Eingetopfte Chinakohlpflanzen (Topfgrösse 10 cm Durchmesser) im 4-Blatt-Stadium werden mit einer wässrigen Wirkstoffemulsion besprüht, die den Wirkstoff in einer Konzentration von 0,75 ppm enthält.

Nach zwei Tagen werden die behandelten Chinakohlpflanzen mit 10 Plutella xylostella-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss Beispiel 1.2. zeigen nach 2 und 5 Tagen 80-100%ige Wirkung (Mortalität).

3.7. Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1.2. zeigen gute Wirkung in obigem Test.

3.8. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 1.2. zeigen gute Wirkung in diesem Test.

3.9. Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1.2. zeigen gute Wirkung im obigen Test.

3.10. Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils so viel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60 % relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1.2. zeigen in obigem Test eine 80-100%ige ovizide Wirkung (Mortalität) gegen Heliothis virescens und Spodoptera littoralis.

## Patentansprüche

1. Verbindungen der Formel I

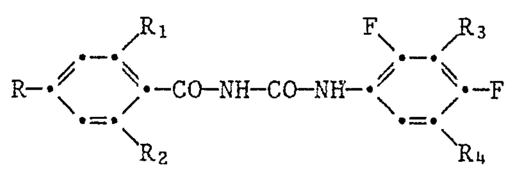

(I),

worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom
stehen.

2. Verbindungen der Formel I gemäss Anspruch 1, worin R für Wasserstoff; $R_1$ für Wasserstoff, Fluor oder Chlor; $R_2$ für Fluor oder Chlor; $R_3$ für Brom und $R_4$ für Chlor oder Brom; oder $R_3$ für Chlor und $R_4$ für Brom stehen.

3. Verbindungen der Formel I gemäss Anspruch 2, worin R für Wasserstoff; $R_1$ für Wasserstoff oder

Fluor; $R_2$ für Fluor oder Chlor; $R_3$ für Brom und $R_4$ für Chlor; oder $R_3$ für Chlor und $R_4$ für Brom stehen.

4. Verbindungen gemäss Anspruch 1 der Formeln

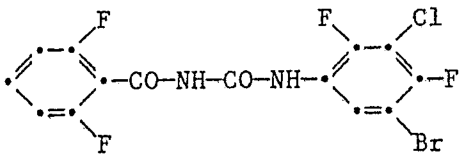

,

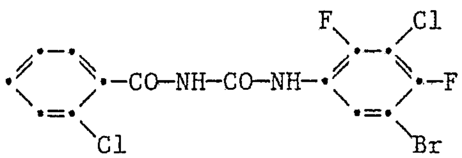

und

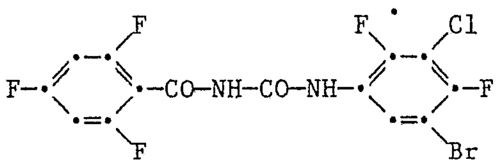

.

5. Verfahren zur Herstellung einer Verbindung der Formel I

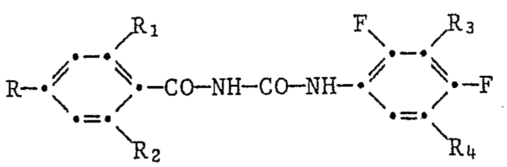

(I),

worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom
stehen, dadurch gekennzeichnet, dass man
a) ein Anilin der Formel II

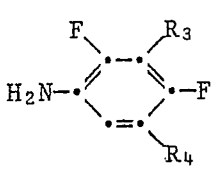

(II)

mit einem Benzoylisocyanat der Formel III

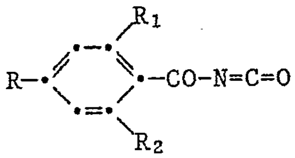

(III), oder

oder

b) ein Isocyanat der Formel IV

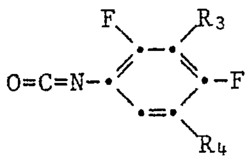

(IV)

mit einem Benzamid der Formel V

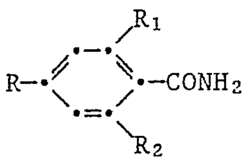

(V), oder

oder

c) ein Anilin der Formel II mit einem Urethan der Formel VI

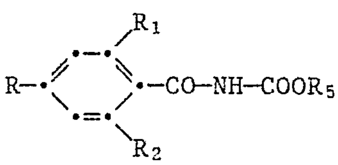

(VI)

umsetzt, wobei in den Formeln II, III, IV, V und VI R, $R_1$, $R_2$, $R_3$ und $R_4$ die angegebene Bedeutung haben und $R_5$ für einen gegebenenfalls halogenierten $C_1$-$C_8$-Alkylrest steht.

6. Schädlingsbekämpfungsmittel, welches als aktive Komponente eine pestizid wirksame Menge einer Verbindung der Formel I

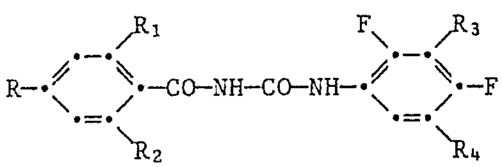

(I)

enthält, worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom

stehen, zusammen mit geeigneten, inerten pflanzenverträglichen Trägern und/oder Zuschlagstoffen.

7. Schädlingsbekämpfungsmittel gemäss Anspruch 6, welches als aktive Komponente eine Verbindung gemäss einem der Ansprüche 1 bis 4 enthält.

8. Verwendung einer Verbindung der Formel I

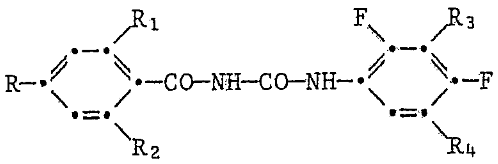

(I),

worin R für Wasserstoff oder Fluor,
$R_1$ für Wasserstoff, Fluor, Chlor oder Methoxy,
$R_2$ für Fluor, Chlor oder Methoxy,
$R_3$ für Brom und
$R_4$ für Chlor oder Brom, oder
$R_3$ für Chlor und
$R_4$ für Brom
stehen, zur Bekämpfung von Schädlingen an Tieren und Pflanzen.

9. Verwendung gemäss Anspruch 8 einer Verbindung der Formel I zur Bekämpfung von Insekten und Arachniden.

10. Verwendung gemäss Anspruch 9 einer Verbindung der Formel I zur Bekämpfung larvaler Stadien von pflanzenschädigenden Insekten.

11. Verbindungen der Formel II

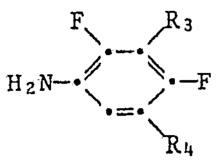

(II),

worin $R_3$ für Brom und
$R_4$ für Chlor oder Brom, oder
$R_3$ für Chlor und
$R_4$ für Brom
stehen.

12. Die Verbindung gemäss Anspruch 11 der Formel

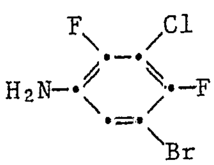

.

13. Verbindungen der Formel VII

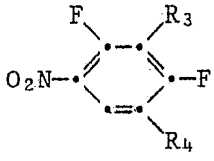

(VII),

worin $R_3$ für Brom und
$R_4$ für Chlor oder Brom, oder
$R_3$ für Chlor und
$R_4$ für Brom
stehen.

14. Die Verbindung gemäss Anspruch 13 der Formel

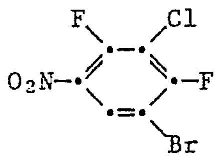

Europäisches Patentamt

# EUROPÄISCHER RECHERCHENBERICHT

Nummer der Anmeldung: EP 88810636.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP - A1 - 0 255 678 (CELAMERCK)<br>* Anspruch 1 *<br>-- | 1,5 | C 07 C 127/22<br>C 07 C 87/60<br>C 07 C 79/12<br>A 01 N 47/34 |
| X | DE - A1 - 3 607 298 (CELAMERCK)<br>* Anspruch 1 *<br>-- | 1 | |
| A | EP - A2 - 0 197 280 (BAYER)<br>* Ansprüche 1,4,6-10 *<br>-- | 1,5,6,<br>8-11 | |
| D,A | DE - A - 2 123 236 (N.V.PHILIPS')<br>* Ansprüche 1,28,76,77 *<br>-- | 1,5,6,<br>11 | |
| D,A | EP - A1 - 0 052 833 (CELAMERCK)<br>* Ansprüche 1-4,7,8 *<br>-- | 1,5,6,<br>8-11 | **RECHERCHIERTE SACHGEBIETE (Int. Cl.4)** |
| A | EP - A1 - 0 035 084 (CIBA-GEIGY)<br>* Ansprüche 1,7-9 *<br>---- | 1,5,6,<br>8-11 | C 07 C 127/00<br>C 07 C 87/00<br>C 07 C 79/00 |

Der vorliegende Recherchenbericht wurde für alle Patentansprüche erstellt.

| Recherchenort | Abschlußdatum der Recherche | Prüfer |
|---|---|---|
| WIEN | 16-12-1988 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument